# EUROPEAN PATENT APPLICATION

(11) **EP 4 198 045 A1**
(43) Date of publication of application: **21.06.2023**
(21) Application number: 21306811.7
(22) Date of filing: 16.12.2021
(51) Int. Cl.: C07K 14/435, A61K 38/00, A61P 31/04

(54) **ANTIMICROBIAL PEPTIDES, VARIANTS AND CHEMICAL ANALOGUES THEREOF AND THEIR USES**

(71) Applicant: Université de Lille, 59800 Lille (FR); CENTRE NATIONAL DE LA RECHERCHE SCIENTIFIQUE (CNRS), 75016 Paris (FR); Université d'Aix Marseille, 13007 Marseille (FR); SORBONNE UNIVERSITE, 75006 Paris (FR); Ecole Centrale de Marseille, 13013 Marseille (FR)
(72) Inventor: TASIEMSKI, Aurélie, 59000 LILLE (FR); WICHLACZ, Céline, 59235 BERSÉE (FR); MARESCA, Marc, 13127 VITROLLES (FR); CANAAN, Stéphane, 13008 MARSEILLE (FR); CAVALIER, Jean-François, 13013 MARSEILLE (FR); MABROUK, Kamel, 13170 LES PENNES-MIRABEAU (FR); HOURDEZ, Stéphane, 29680 ROSCOFF (FR)
(74) Representative: Regimbeau

(57) **Abstract**

The present invention relates to antimicrobial peptides, variants and chemical analogues thereof; in particular, a peptide of 20 amino acids, named Michelicin, having the sequence RVCVRICRNGRCYRRCWNT, derived from a longer precursor with a BRICHOS domain from the marine worm Capitella. The peptide has several cysteines which form disulfide bridges and provide stability in salt conditions. Several derivatives and analogues were produced. The peptide and the derivatives show antimicrobial activity against a wide range of bacteria. The application also concerns nucleic acid sequences encoding these, pharmaceutical composition and to their use as a drug, preservative and disinfectant.

## Description

### TECHNICAL FIELD

The present invention relates to antimicrobial peptides, variants and chemical analogues thereof, to pharmaceutical composition containing any one of them and to their pharmaceutical use and their use as a preservative and disinfectant.

### BACKGROUND OF THE INVENTION

WO2011076605 A discloses antimicrobial peptides isolated from a deep-sea worm called *Alvinella pompejana.* These peptides are salt-resistant and thus can be used in aquaculture production and are active against human pathogens.

Arenicin is an antimicrobial peptide isolated from the coelomocytes of the marine polychaeta lugworm *Arenicola marina.* The article "Solution Structures and Biological Functions of the Antimicrobial Peptide, Arenicin-1 and its linear Derivative" written by Ju-Un Lee and published in "Peptide Science volume 88 number 2 in 2007 shows that the disulphide bridge existing between the two cysteines increases the peptide antimicrobial activity.

Further, the article "Structure and mode of action of the antimicrobial peptide Arenicin" written by J. Andra and published in Biochem. J. (2008) 410 pages 113 to 122 shows that Arenicin is cytotoxic towards Jurkat human cells. The number of multiple-resistance bacterial strains (MRS) is increasing and consequently discovery of novel antimicrobial agents is a need and particularly antimicrobial agent active against MRS.
Moreover, there is also a need for antimicrobial agents which do not induce bacteria resistance.

Moreover, there is a need for non-toxic and non-immunogenic novel antimicrobial agents.

One purpose of the present invention is to provide novel antimicrobial peptides which are non-immunogenic, non-haemolytic and non-cytotoxic.

Another purpose of the present invention is to provide antimicrobial peptides able to treat resistant bacteria strains.

Another purpose of the present invention is to provide antimicrobial agents and particularly peptides which are not immunogenic.

Another purpose of the present invention is to provide a novel preservative, particularly a food or semen preservative.

Another purpose of the present invention is to provide a novel disinfectant which can be used for the disinfection of medical devices, for instance, such as implants or other medical devices particularly medical devices to be implanted in the patient's body.

Another object of the invention is to provide a food composition comprising a peptide according to the invention as a food preservative, for instance.

Another object of the invention is to provide a composition comprising a peptide according to the invention as a semen preservative, for instance.

Another object of the invention is to provide antibiotics for the use in aquaculture.

Another purpose of the present invention is to provide antimicrobial peptides that can be artificially produced (chemical synthesis or recombinant production).

### BRIEF DESCRIPTION OF THE INVENTION

According to one aspect, the present invention relates to an isolated peptide, a variant or a chemical analogue thereof, wherein the isolated peptide and the variant thereof are selected from:
a) peptide consisting in any one of sequences selected from SEQ ID NO: 2 to SEQ ID NO: 12
b) peptide comprising a sequence selected from any one of SEQ ID NO: 2 to SEQ ID NO: 12
c) peptide as defined in a) or b) and comprising a modifying group of its C-terminal carboxyl group and/or of its N-terminal amine group;
d) peptide having at least 80%, particularly at least 90% and more particularly at least 95% identity with peptide as defined in a), b) or c)
e) retro and retro-inverso peptide of peptides as defined in a) to d),
   wherein the chemical analogues are selected from:
f) a chemical analogue consisting in any one of sequences selected from SEQ ID NO: 13 to SEQ ID NO: 19
g) a chemical analogue comprising a sequence selected from any one of SEQ ID NO: 13 to SEQ ID NO: 19
h) a chemical analogue as defined in f) or g) and comprising a modifying group of its C-terminal carboxyl group and/or of its N-terminal amine group,
i) a chemical analogue having at least 80%, particularly at least 90% and more particularly at least 95% identity with analogues as defined in f), g) or h)
j) retro and retro-inverso peptide of peptides as defined in f) to i), said peptide and variants as defined in a) to e) and said chemical analogues as defined in f) to j) having an antimicrobial activity.

According to one embodiment, the isolated peptide, the variants or the chemical analogues thereof may comprise at least two cysteines and particularly four cysteines.

According to another embodiment, the isolated peptide, the variants or the chemical analogues is/are a L-peptide.

According to still another embodiment, the isolated peptide, the variants or the chemical analogues is/are a non-folded peptide.

In addition, the isolated peptide, the variants or the chemical analogues thereof may have a N-terminal modifying group and/or C-terminal modifying group which may be selected independently from each other from acetyl group; formyl group; palmitoyl group; fatty acids groups, particularly myristoyl group, pyroglutamyl group; urea; 7-amino-4-methylcoumarinyl; carbamate; sulphonamide; alkylamine; benzoyl; benzyloxycarbonyl; dye; labels, particularly biotin or fluorescent molecules; NH₂; -O-alkyl,particularly *O*-methyl, *O*-hexyl, *O*-decyl, *O*-tetradecanyl, *O*-methyl and *O*-ethyl;H; NHC₆H₆NO₂(*para*); 7-amino-4-methylcoumarinyl; NHNH₂; NHOH; CH₂Cl; (CH₂)₂NH₂; NHCH₃; NHCH₂CH₃; alkyl and particularly methyl.

According to one embodiment, in the isolated peptide, the variants or the chemical analogues thereof, at least one of cysteines and preferably all cysteines are replaced by an analogue of cysteine and particularly by 2-aminobutanoic group. According to a preferred embodiment, said isolated peptide is of sequence SEQ ID NO 2, wherein at least one of cysteines and preferably all cysteines are replaced by an analogue of cysteine and particularly by 2-aminobutanoic group.

According to another aspect, the present invention further relates to an isolated nucleic acid molecule encoding a peptide, variants or chemical analogues thereof as described above. In particular, the nucleic acid molecule encodes any one of peptides, variants or chemical analogues thereof of SEQ ID NO: 2 to SEQ ID NO: 19. More particularly, the nucleic acid molecule is of sequence SEQ ID NO: 20.

The present invention further relates to a recombinant nucleic acid construct comprising the nucleic acid molecule described above, operably linked to an expression vector.

In addition, the present invention also relates to a host cell comprising the recombinant nucleic acid construct described above, said host cell being particularly from *E. coli* or *P. pastoris.*

According to still another aspect, the present invention also relates to a pharmaceutical composition comprising:
- at least one selected from: the isolated peptide, variants or chemical analogues thereof, the isolated nucleic acid molecule, the recombinant nucleic acid construct and the host cell as described above, and
- a pharmaceutically acceptable excipient.

The isolated peptide, the variants or the chemical analogues thereof, the isolated nucleic acid molecule, the recombinant nucleic acid construct, the host cell or the pharmaceutical composition, as described above may be used as drug.

Particularly, the isolated peptide, the variants or the chemical analogues thereof, the isolated nucleic acid molecule, the recombinant nucleic acid construct, the host cell or the pharmaceutical composition, described above may be used as an antimicrobial agent, particularly an antibiotic for treating a subject in need thereof and more particularly as an antimicrobial agent for treating an infection and/or disease caused by at least one bacterium selected from:
i) Gram negative bacteria, in particular selected from the group of genius consisting of:
   a) *Acinetobacter, particularly Acinetobacter baumannii* (CIP103572) and more particularly *A. baumannii resistant strain (CIP 110431);*
   b) *Salmonella, particularly Salmonella enterica (CIP 80.39)*
   c) *Escherichia, particularly Escherichia coli* and more particularly *E. coli (ATCC 8739), E. coli carbapenem resistant strain (DSMZ 22314) and E. coli (D31);*
   *d) Pseudomonas, particularly Pseudomonas aeruginosa (ATCCC CRM-9027) and more particularly P. aeruginosa fluoroquinolone resistant strain (CIP 107398)*
   *e) Klebsiella, particularly Klebsiella pneumoniae* and more particularly *K. pneumoniae carbapenem resistant strain (DSMZ 26371),*
   *f) Helicobacter, particularly Helicobacter pylori (ATCC 43504);*
   *g) Vibrio, particularly Vibrio alginolyticus and Vibrio diabolicus (HE 800);*
   *h) Burkholderia, particularly Burkholderia cepacia (DSM7288), and*
   *i) Serratia, particularly Serratia marcescens;*
ii) *Gram positive bacteria, in particular* selected from the group of genius consisting of:
   a) *Staphylococcus, particularly Staphylococcus aureus (ATCC 6538P), S. aureus (Pasteur) and more particularly S. aureus (MR, Methicilin Resistant) USA300 (ATCC BAA-1717);*
   *b) Bacillus, particularly Bacillus subtilis (ATCC 6633) and more particularly B. subtilis Nisin resistant (DSM 347) and B. megaterium;*
   *c) Enterococcus, particularly Enterococcus faecalis VRE (Vancomycin Resistant) (DSM 13591);*
   *d) Clostridium, particularly Clostridium perfringens (ATCC 13124), and*
*iii) Mycobacterium species, in particular: M. tuberculosis and more particularly, M. tuberculosis* mc²6230, *M. bovis* BCG *Pasteur, M. abscessus, M. smegmatis, M. ulcerans and M. marinum.*

According to one embodiment, said subject is a mammal, preferably a human being, more preferably a human being suffering from a disease, preferably pulmonary or skin disease.

According to another embodiment, said subject is a fresh or marine animal, preferably selected from the group consisting of:
(i) fishes, preferably carps, cyprinids, and salmonids,
(ii) shellfishes, preferably oysters, clams, cockles, ark shells, scallops and mussels, and
(iii) crustaceans, preferably crabs, lobsters, shrimps and prawns.

According to another aspect, the present invention also relates to a use of the isolated peptide, variants or chemical analogues thereof, the isolated nucleic acid molecule, the recombinant nucleic acid construct or the host cell or the pharmaceutical composition, as described above as preservative for:
(i) *in vitro* treating of a mammal semen
(ii) treating plants, particularly aquatic plants, preferably algae or phytoplankton:

### DETAILED DESCRIPTION OF THE INVENTION

The present invention relates to novel antimicrobial peptides matured from a prepropeptide (also called precursor) with a BRICHOS domain (said peptide corresponding to SEQ ID NO1).

Particularly, the present invention relates to an isolated peptide, variants or chemical analogues therefore as defined above.

More particularly, the peptide of SEQ ID NO: 2 consists in the sequence going from the 209^{th} amino acid residue to the last amino acid residue of a prepropeptide capable of being extracted and purified or being genetically identified from a *Capitella sp* worm and particularly from newly identified *Capitella capitata* (namely *Capitella* C-Channel1 as described in Boidin-Wichlacz *et al* 2021), which are species inhabiting Roscoff, said prepropeptide consisting of SEQ ID NO: 1.

Preferably, the prepropeptide is isolated and purified from *Capitella sp* worm and particularly from *Capitella capitata.*

*Capitella* is a polychaete worm genus in the family *Capitellidae. Capitella* forms a cryptic species complex. There are several already known species of *Capitella sp. Capitella sp* worms live in cohorts regrouping several species that have the same morphology but are genetically distinct and thus cannot reproduce therebetween. The inventors of the present invention found novel species of *Capitella sp* worms (called *Capitella* C-Channel1) that produce novel peptides that can be used as antimicrobial agents (Boidin-Wichlacz et al.,2021). These peptides are secreted and cover the skin of the *Capitella sp* worms, but they can be found inside worms (body fluids, circulating cells as well as the nerve cord). The inventors found a specific prepropeptide of SEQ ID NO: 1. This prepropeptide includes a signal peptide which goes from the 1^{st} to the 23^{rd} amino acid residue of SEQ ID NO: 1. It also includes a pro region with a BRICHOS domain. This pro region goes from the 24^{th} amino acid residue of SEQ ID NO: 1 to the 208^{th} amino acid residue of SEQ ID NO: 1. The last 19 amino acid residues of SEQ ID NO: 1 constitute the peptide called Michelicin, corresponding to SEQ ID NO:2. Michelicin is released through posttranslational modification of the above-mentioned prepropeptide in *Capitella sp* worms. Michelicin and its variants are identified from newly discovered *Capitella sp* from Roscoff (Britania) based on the use of the BRICHOS domain to find new sequences from transcriptomic databases. This constitutes a novel method to identify new antimicrobial peptides.

Thus, another object of the invention is the use of the BRICHOS domain to screen for novel antimicrobial peptide precursors containing novel active peptide sequences, in proteic, DNA or RNA databases. The BRICHOS domain is a molecular chaperone-like domain of 100 amino acids (Stefen et al. 2013) typically present in the prepropeptide sequence of the antimicrobial peptides according to the invention.

As indicated above, one of the peptides of the present invention (called Michelicin) consists in the sequence SEQ ID NO: 2. To the inventors' knowledge, Michelicin is the first antimicrobial peptide found in *Capitella sp* worms. Since Michelicin belongs to a multigenic family and since several species of *Capitellas* share the same habitat, the Inventors found several variants of the above-mentioned prepropeptide. Through analysis of these prepropeptide variants, they determined new peptides that are released after posttranslational modification in *Capitella sp* worms. All the prepropeptide variants include the above-mentioned signal peptide and pro region containing a BRICHOS domain. Accordingly, new potentially antimicrobial peptides can be identified by extracting RNA/DNA from a *Capitella sp* worm, sequencing extracted RNA/DNA and blasting the above-mentioned BRICHOS domain in the obtained libraries. The potentially antimicrobial peptides have the sequence following the BRICHOS domain like Michelicin. Their sequence starts from the 209^{th} amino acid residue to the N-terminal end of the studied prepropeptide sequence.

Besides Michelicin , the Inventors identified 10 variants thereof (called V1-V10 trough the present description). The sequences of Michelicin and these variants are disclosed in Table I.

In addition, the inventors also provided chemical analogues (called CA 1 to CA 7 corresponding to SEQ ID :13 to :19) whose sequence is based on the Michelicin sequences (SEQ ID:2) from *Capitella sp* worms. These chemical analogues are short or modified sequences obtained essentially from the peptide of SEQ ID NO: 2 which are produced by chemical way and not by the *Capitella sp* worms them-self. Therefore, these chemical analogues differ from the peptide variant.

The isolated peptides, the variants and the chemical analogues thereof, subjects of the invention have an antimicrobial activity (as demonstrated by the results shown in Tables III and IV of the examples) against numerous bacterial species , including the so-called "ESKAPE" pathogens (*Enterococcus faecium, Staphylococcus aureus, Klebsiella pneumoniae, Acinetobacter baumanii, Pseudomonas aeruginosa, and Enterobacter species*), a collection of organisms capable of escaping the effects of almost all conventional antibiotics. This group of Gram-positive and Gram-negative bacteria can evade or 'escape' commonly used antibiotics due to their increasing multidrug resistance (MDR). Throughout the world, they are the major cause of life-threatening nosocomial or hospital-acquired infections in immunocompromised and critically ill patients who are most at risk. Among the bacteria sensitive to Michelicin, there are bacteria from genius *Staphylococcus, Mycobacterium and Pseudomonas. Particularly, Staphylococcus aureus, Mycobacterium abscessus Pseudomonas aeruginosa* are known to be commonly responsible of infection in patients suffering from cystic fibrosis (CF). Since the antimicrobial activity of the peptides, the variants and the chemical analogues thereof are preserved even in salty environment (as observed in the lung of patients suffering from CF), they can be used to treat a large amount of pulmonary infections and particularly pulmonary infections dues to the above-mentioned bacteria and therefore to treat specifically patients suffering from cystic fibrosis. They are also active against genius *Burkholderia* and particularly, against *Burkholderia cepacia* which is a bacterial species associated with poor clinical prognosis in CF. These bacteria are highly antibiotic-resistant, and some strains can cause rapid and fatal necrotizing pneumonia.

Moreover, the peptides, the variants and the chemical analogues thereof have also an antimicrobial activity on *Mycobacterium tuberculosis* which causes tuberculosis in Human.

The peptides, the variants and the chemical analogues thereof, subjects of the present invention have also an antimicrobial activity on *Helicobacter, particularly on Helicobacter pylori* a causative agent of chronic inflammation associated with a strong risk of developing duodenal/gastric ulcer disease and gastric cancer. Interestingly, the peptides, the variants and the chemical analogues thereof are resistant to stomach enzymes.

They are also active against *Vibrio sp.,* particularly against *Vibrio alginolyticus* which is a marine bacterium causing infection in humans leading to otitis and to wound infection. *V. alginolyticus* causes also severe infection in shellfish hatcheries as well as in humans after eating infected adults of oyster, mussel, clam, scallop and crab. Owing to their resistance to salty environment, the peptides, the variants and the chemical analogues thereof of the present invention can also be used to treat living marine organisms.

In addition, the peptides, the variants and the chemical analogues thereof may be used as a food preservative. They can be combined with salt; salt is a flavour exhauster and a food preservative agent too. Owing to their food preservative activity, they can replace salt. Salt is often used as a food preservative whereas it is known to cause stomach cancer and blood hypertension. Peptides of the invention can be advantageously used in delicatessen in combination with salt or to replace it, at least partially.

Moreover, the salt resistance of the peptide, the variants and the chemical analogues thereof of the present invention enables a topical use thereof since they will have an antimicrobial activity even on a sweating skin. They can also be used in cosmetic preparation such as deodorant.

The peptides, the variants and the chemical analogues thereof, subjects of the present invention also have a bactericidal effect against *Serratia genius,* particularly against *Serratia marcescens,* now recognized as an important opportunistic pathogen combining a propensity for healthcare-associated infection and antimicrobial resistance in Human. Bacterial contamination by *Serratia marcescens* is also a serious and very real deterrent to profitable semen production in swine. It is routinely observed in raw and extended semen produced for artificial insemination when semen is collected by the gloved-hand technique. Adding to their activities against *Serratia,* the variants and the chemical analogues thereof, subjects of the present invention are not cytotoxic on spermatozoids.

Further, the antimicrobial activity of the peptides, the variants and the chemical analogues thereof is preserved even at 90°C. The peptides of the invention are active against microorganisms at 37°C and more and can be thus used in pasteurized food or can food and do not need any peculiar storages to keep their properties. The thermal resistance of the peptides of the present invention was very surprising for the inventors since *Capitella sp* worms and particularly *Capitella capitata* inhabit in various habitat and not particularly in hot environment/habitat.

According to one embodiment, the chemical analogues CA 1 to CA 7 (SEQ ID NO: 13 to SEQ ID No: 19 are smaller than natural peptides and its variants (SEQ ID NO: 2 to SEQ ID NO: 12) and can be easily produced by chemical synthesis such as Fmoc solid phase synthesis (described in the examples).

Preferably, the peptides, the variants and the chemical analogues thereof, subjects of the invention are linear (*i.e*.: not branched-peptides or cyclic). Preferably, they are L-peptides.

In addition, they can be synthesized in their D form and with an amidation to increase their resistance to proteases.

The aforementioned peptides, the variants and the chemical analogues thereof can be folded or non-folded. When they are folded, they comprise at least one or two disulphide bridges between two cysteines.

In a preferred embodiment, the peptide, the variants and the chemical analogues thereof comprises at least one disulphide bridge and particularly 2 disulphide bridges. They are still active as antimicrobial agents even when they comprise only one disulphide bridge and even no disulphide bridge. Their activity and/or their thermal stability seem(s) to be greater when comprising at least one disulphide bridge and more advantageously two disulphide bridges. However, surprisingly, peptides with two cysteines and more particularly four cysteines, (these peptides are also defined here as linear non-folded analogues) keep their antimicrobial properties (results demonstrated in table VI of the examples. These peptides can be produced more easily and are economically interesting alternatives to Michellicin and the other peptides of the present invention.

In a preferred embodiment, the peptides of the invention are non-folded peptides.
The present invention also relates to chemical compound as above defined wherein at least one cysteine and preferably all the cysteines are replaced by a chemical analogue of cysteine and particularly by 2-AminoBUtanoic group (ABU) and particularly an isolated peptide of sequence SEQ ID NO 2 wherein at least one cysteine and preferably all the cysteines are replaced by an analogue of cysteine and particularly by 2-aminobutanoic group (referred here as ABU form of the peptides).

The peptides of the present invention can be isolated and purified from *Capitella sp* worms and particularly from *Capitella capitata* according to any methods known by the those skilled in the art.

The modifying C-terminal modifying group and/or the N-terminal modifying are chosen independently one from the other among : acetyl group, formyl group, palmitoyl group (and other fatty acids), myristoyl group, pyroglutamyl group, urea, 7-amino-4-methylcoumarinyl, carbamate, sulphonamide, alkylamine, benzoyl, benzyloxycarbonyl, biotin, dye and other fluorescent molecules, NH₂, -*O*-alkyl and particularly *O*-methyl, *O*-hexyl, *O*-decyl, *O*-tetradecanyl, *O*-methyl and *O*-ethyl, H, NHC₆H₆NO₂(*para*), 7-amino-4-methylcoumarinyl, NHNH₂, NHOH, CH₂Cl, (CH₂)₂NH₂, NHCH₃, NHCH₂CH₃, alkyl and particularly methyl.

The present invention also relates to an isolated nucleic acid molecule encoding a propeptide and a peptide according to the invention. In particular the nucleic acid molecule encodes the SEQ ID NO: 1. More particularly, the nucleic acid molecule encodes a peptide selected from peptides of SEQ ID NO: 2 to SEQ ID NO: 12 or chemical analogues thereof of SEQ ID NO: 13 to 19. Even more particularly, the nucleic acid molecule is of SEQ ID NO: 20.
The present invention also related to a recombinant nucleic acid construct comprising the nucleic acid molecule according to the invention, operably linked to an expression vector.

The peptides and the variants of the invention can be produced in eukaryote and prokaryote cells. DNA sequence coding various peptides of the invention can be synthetized and cloned into specific plasmids commercially available to produce peptides in *E. coli* as well as *Pichia pastoris.* Recombinant plasmids can be used to transform T7 Express Competent *E. coli* strain as well as X33 *P. Pastoris* competent yeast cell. After production, peptides are purified and lyophilized as commonly known.

Accordingly, the present invention also relates to a host cell comprising the recombinant nucleic acid construct of the invention, said host cells being particularly chosen among *E. coli* and *P. pastoris* but not limited thereto.

The present invention relates to an isolated peptide according to the invention for its use as a drug and particularly as an antimicrobial agent to treat a subject in need of such a treatment and more particularly as an antimicrobial agent in the treatment of an infection and/or an infectious disease caused by at least one bacteria chosen among:
i) Gram negative bacteria such as : *Acinetobacter baumannii (*CIP 103572) and particularly *A. baumannii* antibiotic-resistant strain *(CIP 110431), Salmonella enterica (CIP 80.39), Escherichia coli* and particularly *E. coli (ATCC* 8739*), E. coli* carbapenem-resistant strain *(DSMZ 22314), E. coli (D31), Pseudomonas aeruginosa (ATCCC CRM-9027) and particularly P. aeruginosa FQR (FluroQuinolone Resistant) (CIP 107398), Klebsiella pneumoniae* and particularly *K. pneumoniae* carbapenem-resistant strain *(DSMZ 26371), Helicobacter pylori (ATCC 43504), Vibrio alginolyticus and V. diabolicus (HE 800), Burkholderia cepacia (among which DSM 7288) and other Burkholderia sp., and Serratia marcescens (among which 231, BO71).*
ii) *Gram positive bacteria such as: Staphylococcus aureus (ATCC 6538P), S. aureus (Pasteur) and particularly S. aureus (MR, Methicilin Resistant) USA300 (ATCC BAA-1717), Bacillus subtilis (ATCC 6633) and particularly B. subtilis Nisin resistant (DSM 347), B. megaterium, Enterococcus faecalis VRE (Vancomycin Resistant) (DSM 13591), Clostridium perfringens (ATCC 13124).*
iii) *Mycobacterium species such as : M. tuberculosis and particularly, M. tuberculosis mc²6230 M. bovis BCG Pasteur, M. abscessus, M. smegmatis, M. ulcerans, M. marinum.*

The correspondence between the infectious agent and the name of the disease is well known from those skilled in the art.

The traded subject is not limited according to the present invention. The subject can be selected from plants like aquatic plants such as algae and phytoplankton, trees and particularly chestnut tree, mammals and in particular human or pigs, fresh water and saltwater aquatic organisms, particularly fishes like carps, salmon and other cyprinids and salmonids, shellfish, molluscs such as oysters, clams, cockles, ark shells, scallops and mussels, crustaceans such as crabs, lobsters, shrimps and prawns. In addition, the subject may be a subject who/which has already received an antibiotic treatment for treating an infection and/or a disease caused by the one of the above-mentioned microorganisms.

The present invention relates to a pharmaceutical composition comprising at least one isolated peptide, variant or chemical analogue, or a nucleic acid encoding the peptides and the variant or a nucleic acid construct according to the invention and a pharmaceutically acceptable excipient. The pharmaceutically acceptable excipient is not limited according to the invention. It can be selected from well-known pharmaceutically acceptable excipients such as those disclosed in Rowe et al, eds Handbook of Pharmaceutical Excipients, 7th edn, London, pharmaceutical press 2012. The excipient is chosen according to the administration way of the pharmaceutical composition of the invention.

The effective amount of isolated peptide, variants or chemical analogues of the invention can be determined by the person skilled in the art in relation with the subject to be treated, to the bacteria causing infection and the route of administration, for example.
According to one embodiment, the pharmaceutical composition of the invention also comprises a nanoparticle or a particle playing the same role able to enhance the antimicrobial activity of the isolated peptide of the invention.

The route of administration of the pharmaceutical composition of the invention is not limited. It can be oral, enteral/gastrointestinal, topical, rectal, parenteral (intramuscular injection or intra venous injection), nasal administration, inhalation (especially for pulmonary infections) or nebulization, for example.

The form of the pharmaceutical composition of the present invention is not limited. It can be tablets, capsules, liquid, nasal spray, spray for inhalation, cream or gel, for example.

The present invention also relates to a disinfectant comprising at least one selected from isolated peptide, variant or chemical analogue of the invention, the isolated nucleic acid molecule, the recombinant nucleic acid construct or the host cell, as described above. This disinfectant can be used for the disinfection of medical devices, particularly for disinfection of medical devices that can be inserted into a patient's body.

The present invention further relates to a food preservative comprising at least one selected from isolated peptide, variant or chemical analogue according to the invention, the isolated nucleic acid molecule, the recombinant nucleic acid construct or the host cell, as described above. This food preservative can replace at least partially salt and/or nitrites which are suspected to cause cancer.

The present invention relates to a food composition particularly a food composition chosen among pasteurized food compositions, salted food compositions like salted meat, canned food compositions and fermented food compositions, and comprising at least one selected from isolated peptide, variant or chemical analogue according to the invention, the isolated nucleic acid molecule, the recombinant nucleic acid construct or the host cell, as described above.

The present invention further relates to a semen preservative comprising at least one selected from isolated peptide, variant or chemical analogue according to the invention, the isolated nucleic acid molecule, the recombinant nucleic acid construct or the host cell, as described above.

The terms used in the description above are defined hereunder for facilitating the comprehinsion of the present invention.
*Capitella sp* worm refers particular to a worm chosen among *Capitella aberranta, Capitella capitata, Capitella caribaeorum, Capitella dizonata, Capitella giardi, Capitella gracilis, Capitella hermaphrodita, Capitella intermedia, Capitella jonesi, Capitella minima, Capitella ovincola, Capitella perarmata, Capitella perarmatus, Capitella singularis, Capitella teleta* and *Capitella tripartita.*
AMP refers to an antimicrobial peptide.

The term "peptide" englobes, unless specified, folded and non-folded peptides (i.e. having at least one disulphide bond between two cysteines) and the optically active forms of said peptide. A peptide is a continuous amino acid chain of amino acids bonded together with covalent liaisons. The term "peptide" refers to a small protein when said continuous amino acid chain of less than 50 amino acids, preferably of less than 40, more preferably of less than 30 or less than 20 amino acids. Particularly, the peptide sequences comprise between 5 and 50 amino acids, more particularly, between 10 and 40 and even more particularly, between 15 and 30 amino acids.

In the present description the term "peptide" may also refer to the peptide variants which are produced by the same life organism as the wild peptide.

A L-peptide consist of a chain of L-amino acids which are amino acids in their natural/common form. Reflecting the L-amino acids by a mirror are their enantiomers, D-amino acids, which share identical chemical and physical properties of L-amino acids, except for their ability to rotate plane-polarized light in opposite directions. Application of D-amino acids enhances biostability of peptide therapeutics.

Consequently, a D-peptide consists of a D-amino acids chain. A D-peptide of a given sequence is a chain of D-amino acids corresponding to said sequence. A D peptide can also be called according to the invention an "inverso peptide". According to the invention, a retro peptide is a peptide in which the direction of the sequence is reversed compared with a parent peptide. In other words, the C-terminal of the parent peptide is the N-terminal of the retro peptide and the N-terminal of the parent peptide is the C-terminal of the retro peptide.

According to the invention a retro-inverso peptide is a retro peptide constituted of D-amino acids.

"Antimicrobial activity" refers to growth inhibition and/or population decrease in a microorganism chosen among virus, bacteria and fungi obtained when submitted to an agent under given conditions which are not restrictive. Particularly, in the context of the present invention, an antimicrobial agent is an agent having a bactericidal or bacteriostatic effect. More particularly, the antimicrobial agent is an antibiotic.

"Percentage of identity" refers to the percentage of amino acid residues or nucleotides identical between a sequence to be compared and a reference sequence. Said percentage is determined through a comparison window and is obtained with the best alignment between the sequence to be compared and the reference sequence. Said alignment can be total (the reference sequence and the sequence to be compared are aligned) or only one part of reference sequence (the comparison window) is aligned with one part of the sequence to be compared. The peptides having a given % of identity can derive from the reference sequence by substitution and/or suppression and/or addition of one or several amino acids in the reference sequence, and/or by modification of at least one CONH peptide linkage, particularly by introduction of a retro or retro-inverso type linkage and/or by substitution of at least one amino acid particularly with a non-proteinogenic amino acid chosen among carnitine, L-canavanine, L-DOPA, hydroxyproline, 2-aminobutanoic group and seleno-methionine. The percentage of identity can be determined using computer software like BLAST (PHI-BLAST, BLAST N, BLAST P), FASTA or BESTFIT, for example. Unless specified, the comparison window refers to the entire sequence of the reference sequence.

In the present application, the peptide sequences are written from N-terminal to C-terminal.

The term "modifying group" englobes all the groups that can be covalently linked to peptide C-terminal or peptide N-terminal. As regards N-terminal modifying group, the following groups can be linked to the N atom of the N-terminal. The following groups are given as non-limiting examples: acetyl group, formyl, palmitoyl group and other fatty acids as for example myristoyl group, pyroglutamyl group, urea, 7-amino-4-methylcoumarinyl, carbamate, sulphonamide, alkylamine, benzoyl, benzyloxycarbonyl, biotin, dye and other fluorescent molecules.

As regards C-terminal modifying groups, the following groups can be given as non-limiting examples for replacing the OH-radical of the carboxyl group: NH₂, -*O*-alkyl and particularly *O*-methyl, *O*-hexyl, *O*-decyl, *O*-tetradecanyl, *O*-methyl and *O*-ethyl, H, NHC₆H₆NO₂(para), 7-amino-4-methylcoumarinyl, NHNH₂, NHOH, CH₂Cl, (CH₂)₂NH₂, NHCH₃, NHCH₂CH₃, alkyl and particularly methyl. Groups cited herein above as referred to the N-terminal modification can also be used to modify the C-terminal.

The terms "operably linked" is meant than the aforesaid nucleic acid molecule is covalently linked to an expression vector enabling ribosome to translate said nucleic acid molecule or permitting RNA polymerase to produce an mRNA encoding said peptide.
An expression vector is a molecule consisting of nucleic acids, in particular a plasmid, which comprises a promoter region and optionally an enhancer region. The expression vector can include other genes such as antibiotic resistance gene in order to select host cells that contain the nucleic acid molecule encoding the claimed peptide.

A host cell is a living cell, in particular a bacterial cell or a eukaryotic cell or a non-living medium comprising transcriptional and/or translational machinery and optionally organelles from a cell, permitting to amplify a given nucleic acid construct and/or produce a given peptide.

A nucleic acid molecule is a single or double DNA chain or a single RNA chain including DNA/RNA hybrid which encodes for the corresponding peptide or for the corresponding prepropeptide.

The terms "fermented food" refers to beverage or solid food obtained by fermentation of a bacteria or a yeast; fermented food comprises fermented beverage like wine, cider, beer, kefir, fermented milk, yoghurt, as well as cheese, lacto-fermented vegetables, like olives, for example.

The term "treatment" shall be interpreted as a curative treatment as well as a prophylactic treatment.

The terms "peptide of the invention "or "peptide according to the invention" refer to isolated peptides as described above.

### FIGURES

Fig. 1 shows growth of *V. alginolyticus* (%) *vs* incubation time (in min) at 90°C when *V. alginolyticus* cultures are treated with Michelicin, Arenicin or Alvinellacin, respectively;
Fig. 2 shows % of haemolysis *vs.* concentration (µM) of Michelicin, Alvinellacin or Arenicin;
Fig. 3 shows the biofilm formation measured by OD₅₉₅ for untreated cultures of *Pseudomonas* or *V. diabolicus,* and for *Pseudomonas* or *V. diabolicus* cultures treated with 20 µM Michelicin, 20 µM Arenicin and 20 µM Alvinellacin, respectively.
Fig. 4 shows the % of viability of BEAS cells treated for 48 h with Michelicin or Arenicin *vs.* the concentration in Michelicin or Arenicin in µM;
Fig. 5 shows the % of viability of HUVEC cells treated for 48 h with Michelicin or Arenicin *vs.* the concentration in Michelicin or Arenicin in µM;
Fig. 6 shows the fold increase in initial MIC *vs.* time in days for *P.aeruginosa* submitted to continuous exposure to sub-lethal doses of an antibiotic chosen among Michelicin, Imipenem and Gemifloxacin;
Fig. 7 and Fig. 8 show the fluorescence of propidium iodide expressed in RFU (relative fluorescence unit) *vs.* time (min) obtained when treating *P. aeruginosa* with several compounds at 5-times their MIC (including Michelicin, other known AMPs and detergent (*i.e.,* CTAB)) able to perforate the bacteria cell membrane by pore-forming activity;
Fig. 9 shows scanning electron microscopic images of *V. alginolyticus* (first line) and *V. diabolicus* (second line) without treatment (first image of each line), treated with Michelicin (second image of each line) and Alvinellacin (third image of each line).
Fig. 10 shows the fluorescence of propidium iodide expressed in RFU (relative fluorescence unit) *vs.* the concentration of Michelicin (in µM) obtained when treating *V. alginolyticus* or *V. diabolicus* with increasing concentration of Michelicin at 37° C during 5 min.

### EXPERIMENTAL PART

### I. Determination of Michelicin and its variants

- **Animal collection and RNA extraction:** *Capitella sp* were collected at low tide at two different sites: the old harbor in Roscoff and the beach called Le Laber, near Roscoff. The fine sediment was sieved on a 500 µm mesh in the field and the result was brought back to the laboratory for sorting under a dissecting microscope. For each population, 30 worms were placed in RNA-later and the total RNAs were extracted with the TRI-Reagent solution (Sigma), following the manufacturer's protocol. The RNAs were re-suspended in DEPC-treated water and the quality and quantity were evaluated on a Nanodrop.
- **Sequencing:** The transcriptome sequencing was performed at Genoscreen (Lille, France). An Illumina library was prepared for each on the three populations. The proportion of mRNA was enriched by retention on oligod(T) beads. Each library was sequenced on one lane of HiSeq 2000 (100 million clusters, 2x100 bases paired-end).
- **Assembly and determination of the abundance of assembled contigs:** The analyses were all carried out in the Galaxy environment and the computing power was provided by the ABiMS platform (Station Biologique de Roscoff, France). The 100-base sequences for each population were first filtered for quality with Prinseq-lite, and the pairs of sufficient quality sequences were established (GetPairs). Reads (typically about 25 million paired reads per library) were assembled with Trinity after normalization to reduce the size of the dataset. This was performed for the two libraries and the resulting contigs were concatenated. Redundancy was removed with CAP3. This final assembly of mRNA sequences in the libraries was then used as a template to blast the BRICHOS domain.
- ***Molecular identification of Michelicin and its genetic variants (paralogs and orthologs):*** Michelicin is naturally processed from a protein precursor named preproAMP constituted by a signal peptide, a pro region with a BRICHOS domain and the AMP at the C-terminal part of the protein precursor. Surprisingly, the BRICHOS domain is well conserved between *Capitella sp* worms. The nucleotide sequence coding the prepromichelicin precursor and its variants were obtained by blasting the amino acid sequence of the BRICHOS domain comprised in SEQ ID NO: 1 to the contigs of transcriptomic databases obtained by RNA sequencing.

SEQ ID NO: 1:
MEKVLIQDQPPKYGAGRTDTKKTCLLMYAVAMTILVFAIMVTGVVMFVLHVDNKVDIDTLPSTVEHY
QVNGEDIEQHVLIDDARETEIIRYEDSGAILVLDYRKGLTALYVPSAKECFLTGGIDRNLPSPFHGEVE
KNDAIDDGAEVTYRKCNSFPVMDHSVLPPHLTSFCEHKPVFWVVPSNGDQGEQRTKRSPGRVCVRI
CRNGRCYRRCWNT

The amino acid sequence of Michelicin and its variants (V1 to V10) are recorded in Table I as well as the chemical (synthetic) analogues of the peptides of the invention (SEQ ID 11 to 19).

**Table I**

| *Name* | Sequences | *SEQ ID NO* |
|---|---|---|
| *Michelicin* | RVC₁VRIC₂RNGRC₃YRRC₄WNT With disulphide bridges between C1 and C4; C2 and C3 | 2 |
| *V1* | RVC₁VRVC₂RNGRC₃YRRC₄WNT With disulphide bridges between C1 and C4; C2 and C3 | 3 |
| *V2* | RFC₁VRVC₂RNGRC₃IMKC₄WNT With disulphide bridges between C1 and C4; C2 and C3 | 4 |
| *V3* | RIC₁VRVC₂RNGRC₃IMKC₄WNT With disulphide bridges between C1 and C4; C2 and C3 | 5 |
| *V4* | RIC₁VRVC₂RNGFC₃FMKC₄WNT With disulphide bridges between C1 and C4; C2 and C3 | 6 |
| *V5* | RFC₁VRVC₂LFIRC₃FMKC₄WNT With disulphide bridges between C1 and C4; C2 and C3 | 7 |
| *V6* | RIC₁VRVC₂RNGRC₃FMKC₄WNT With disulphide bridges between C1 and C4; C2 and C3 | 8 |
| *V7* | RKAVSTC₂VRVC₃RNGFC₄FMKCWNT With disulphide bridges between C1 and C4; C2 and C3 | 9 |
| *V8* | RVC₁VRIC₂RNGFC₃FMKC₄WNT With disulphide bridges between C1 and C4; C2 and C3 | 10 |
| *V9* | RVC₁VRVC₂RNGFC₃FMKC₄WNT With disulphide bridges between C1 and C4; C2 and C3 | 11 |
| *V10* | RVC₁IRVC₂RNGVC₃YRRC₄WG With disulphide bridges between C1 and C4; C2 and C3 | 12 |
| CA1 | KVC₁VKIC₂KNGKC₃YKKC₄WNT With disulphide bridges between C1 and C4; C2 and C3 | 13 |
| CA2 | CRNGRC | 14 |
| CA3 | CRNGRCYRR | 15 |
| CA4 | RICRNGRCYRR | 16 |
| CA5 | CVRICRNGRCYRRC | 17 |
| CA6 | SEQ ID NO: 2 wherein all the cysteines are replaced by Abu | 18 |
| CA7 | SEQ ID NO: 2 wherein all the cysteines are replaced by Abu and having a N terminus linked with a cycle | 19 |

SEQ ID NO: 20 (5' cgcgtctgtgttaggatttgtcgcaatggacggtgctacagaagatgttggaacact 3') is the DNA sequence encoding for Michelicin.

### Peptide synthesis

Michelicin (SEQ ID NO: 2) its variants (V1 to V10, SEQ ID 3 to SEQ ID 13) and its chemical analogues (SEQ ID 11 to 19) were synthesized as follows.

4-hydroxymethyl-phenoxymethyl-co-polysterene-1%divinylbenzene resin (HMP resin), Fmoc-Rink amide resin, N- fluorenylmethyloxycarbonyl (Fmoc) amino acid derivatives were purchased from Perkin-Elmer. All solvents were analytical-grade commercial product from Perkin Elmer or SDS (Peypin, France). The aforementioned peptides were classically chemically produced by the solid-phase method. Stepwise elongation of the Michelicin and analogs was carried out on 0.35 mmol of HMP resin (0.96 mmol of hydroxyl site) or Fmoc-Rink amide resin using an automatic synthesizer (Model 433A, Applied Biosystems Inc.) To obtain short analogs aliquots of peptide-resin were removed at their corresponding cycle. Trifunctional amino acids were side chain protected as follows: trityl (Trt) for Cys, His and Asn; t-butyl (t-Bu) for Thr and Tyr; Boc for Lys and Trp, and pentamethylchloroman (Pmc) for Arg. N--amino group were deprotected by treatment with 18% and 20% piperidine/N-methylpyrrolidine for 3 and 8 min, respectively. The Fmoc-amino acid derivatives were coupled (20 min) as their hydroxybenzotriazole active ester in N-methylpyrrolidone (four folds excess). After peptide chain assembly, the peptidyl resin was cleaved and deprotected by a 2 h treatment at 25°C with TFA containing 5% thioanisole 5% ethanedithiol in a final volume of 10 mL/g of peptidyl resin. The reduced peptides were dissolved at a concentration of 1 mg/mL in 0.2 M Tris-HCl buffer (pH 8) 30% acetonitrile (v/v) and stirred under air to allow folding (48 h, 25°C). The oxidation process was monitored every 60 min by analytical reversed-phase HPLC using a 60 min linear gradient of 0 to 60% acetonitrile in 0.1% (by vol.) TFA/H₂O at flow rate of 1 mL/min (UV detection=214 and 280 nm). The eluate was filtered and purified to homogeneity by preparative reversed-phase HPLC (Perkin Elmer, ODS 20 um, 100 x 10 mm) using a 2h linear gradient of 0 to 60% acetonitrile in 0.1% TFA/H₂O (A) and 90% acetonitrile in water containing 0.1% TFA (B) at a flow rate of 6 mL/min, with UV detection at 214 nm. The chemical identity of the synthetic peptides was determined by electrospray mass spectrometry and found to be consistent with the calculated mean M.W. of peptides.

### II. Physicochemical properties of Michelicin and its variants

Chemical parameters, isoelectric point and net charge at pH=7 were determined by using the Analysis Tool - Peptide 2.0 PepCalc.com - Peptide calculator. The calculated values are recorded in Table II.

**Table II (herein above)**

| *Name* | *Chemical parameters* | *Isoelectric point (PI)* | *Net charge (pH 7)* |
|---|---|---|---|
| *Michelicin* | *Hydrophobic: 21.05%* | 11.81 | 5.9 |
| | *Acidic: 0%* | | |
| | *Basic:31.58%* | | |
| | *Neutral: 47.37%* | | |
| *V1* | *Hydrophobic: 21.05%* | 11.81 | 5.9 |
| | *Acidic: 0%* | | |
| | *Basic: 31.58%* | | |
| | *Neutral: 47.37%* | | |
| *V2* | *Hydrophobic: 31.58%* | 11.67 | 4.9 |
| | *Acidic: 0%* | | |
| | *Basic: 26.32%* | | |
| | *Neutral: 42.11%* | | |
| *V3* | *Hydrophobic: 31.58%* | 11.67 | 4.9 |
| | *Acidic: 0%* | | |
| | *Basic: 26.32%* | | |
| | *Neutral: 42.11%* | | |
| *V4* | *Hydrophobic: 31.58%* | 11.67 | 4.9 |
| | *Acidic: 0%* | | |
| | *Basic: 26.32%* | | |
| | *Neutral: 42.11%* | | |
| *V5* | *Hydrophobic: 47.37%* | 11.11 | 3.9 |
| | *Acidic: 0%* | | |
| | *Basic: 21.05%* | | |
| | *Neutral: 31.58%* | | |
| *V6* | *Hydrophobic: 31.58%* | 11.67 | 4.9 |
| | *Acidic: 0%* | | |
| | *Basic: 26.32%* | | |
| | *Neutral: 42.11%* | | |
| *V7* | *Hydrophobic: 34.78%* | 11.28 | 4.9 |
| | *Acidic: 0%* | | |
| | *Basic: 21.74%* | | |
| | *Neutral: 43.48%* | | |
| *V8* | *Hydrophobic: 36.84%* | 11.11 | 3.9 |
| | *Acidic: 0%* | | |
| | *Basic: 21.05%* | | |
| | *Neutral: 42.11%* | | |
| *V9* | *Hydrophobic: 36.84%* | 11.11 | 3.9 |
| | *Acidic: 0%* | | |
| | *Basic: 21.05%* | | |
| | *Neutral: 42.11%* | | |
| *V10* | *Hydrophobic: 27.78%* | 11.52 | 4.9 |
| | *Acidic: 0%* | | |
| | *Basic: 27.78%* | | |
| | *Neutral: 44.44%* | | |

Based on the values shown in Table II, Michelicin and its variants seem to be highly soluble in water and salt water solutions at pH=7 since their isoelectric point is at pH around 11. Further, Michelicin and its variants are hydrophobic. This hydrophobicity enables them to interact with the lipidic membrane of cells. Further, Michelicin and its variants are cationic suggesting that they can interact with the cell membrane and form pore therein. This mechanism which explains at least partly the antimicrobial activity of Michelicin, and its variants will be shown herein after.

### III. Biological activity of the peptides

### 1) Antimicrobial activity test in the presence or absence of salt and comparison with Alvinellacin and Arenicin

Alvinellacin and Arenicin are already known antimicrobial peptides isolated from *Alvinella sp* sea worm and lugworm, respectively.

To standardize the bacterial cell suspension for assay of antibacterial activity, some colonies of each strain grown overnight on MHA (Mueller Hinton Agar. ROTH) plates were suspended in MHB (Mueller Hinton Broth. ROTH) or MHB saline (Mueller Hinton Broth containing 565 mM NaCl) and incubated at 37°C or 30°C until visible turbid. This log-phase inoculum was diluted 1: 100 in the same culture media. Using sterile 96-well microtiter plates (CELLSTAR. Greiner bio-one), serial 2-fold dilutions

(40 to 0.78 µM) of each peptide were prepared in cation-adjusted acetic acid 0.02% with 0.4% BSA (Bovin Serum Albumin fraction V. Boehringer) at a volume of 10 µL/well. Each well was inoculated with 100 µL of the standardized bacterial inoculum, corresponding to a final test concentration of about 10⁵ CFU/mL. Antimicrobial activities were expressed as the MIC (Minimal Inhibitory Concentration), the lowest concentration of peptide at which 100% inhibition of microbial growth was observed after 24 h of incubation at 37°C or 30°C by measuring OD₆₀₀ in a microtiter plate reader. The minimal bactericidal concentration (MBC) was defined as the peptide concentration where no colony growth was observed.

The results are recorded in Table III

**Table III**

| **Bacterial strains** | **MIC (µM)** | **Michelicin** SEQ ID NO:2 | **Alvinellacin** | **Arenicin** |
|---|---|---|---|---|
| *Vibrio alginolyticus* | MIC | 0.07-0.15 | 0.07-0.15 | 0.15-0.31 |
| | MIC with 565mM NaCl | 0.62-1.25 | 0.07-0.15 | 0.07-0.15 |
| *V. alginolyticus (MPV19* IFREMER) | MIC | 0.07-0.15 | 0.07-0.15 | 0.15-0.31 |
| | MIC with 565mM NaCl | 5-10 | 0.62-1.25 | 2.5-5 |
| *V. diabolicus* | MIC | 2.5-5 | 2.5-5 | 1.25-2.5 |
| | MIC with 565mM NaCl | >40 | 2.5-5 | 10-20 |
| *Pseudomonas sp.* | MIC | 2.5-5 | 1.25-2.5 | 2.5-5 |
| | MIC with 565mM NaCl | 20-40 | 5-10 | 2.5-5 |
| *Bacillus megaterium* | MIC | 0.03-0.07 | 0.15-0.31 | 0.07-0.15 |
| | MIC with 565mM NaCl | 10-20 | 2.5-5 | 20-40 |

The results of Table III show that Michelicin is an efficient antimicrobial agent against *Vibrio alginolyticus* responsible of otitis and wound infections, *Pseudomonas, B. megaterium* and even *V. diabolicus.* Accordingly, Michelicin can be useful for treating various common gram-negative bacteria and related diseases. Michelicin has an antimicrobial activity which, surprisingly, is not limited to marine bacteria. For instance, *Pseudomonas* is commonly found in human and plants as a pathogenic agent. *Pseudomonas* is also commonly found in hospitals and then often infects medical devices. Accordingly, Michelicin and its variants can also be used for the disinfection of medical devices such as implants, stents or any other medical devices.

Moreover, these results show that Michelicin is also active when the environment is salty. Accordingly, Michelicin can be used in aquaculture of for food conservation, especially for pasteurized food, fermented food or can food. Can food (pasteurized or fermented food) contain salt as a conservative agent and may be further submitted to high temperature treatment (pasteurization).

### 2) Antimicrobial activity of Michelicin on various mycobacterial strains and even multi drug resistant strains and comparison with Alvinellacin and Arenicin

The same experiments as described in 1) were performed. For the evaluation of antimicrobial activity against some other strains, a resazurin microtiter assay was used as explained hereinafter.

### Resazurin microtiter assay (REMA) for MIC (Minimum Inhibitory Concentration) determination.

Susceptibility testing against the various mycobacterial strains was performed using the Middlebrook 7H9 broth microdilution method. All assays for each strain were carried out at least in triplicate. MICs of the peptides, were determined in 96-well flat-bottom Nunclon Delta Surface microplates with lid (ThermoFisher Scientific, ref. 167008) using the resazurin microtiter assay. Briefly, log-phase bacteria were diluted to a cell density of 5 × 10⁶ cells/mL in 7H9-S^{OADC} (7H9 broth + 10% OADC + 0.2% glycerol + 0.05% Tween 80, and 24 µg/mL D-panthothenate when needed). Then 100 µL of the above inoculum (*i.e.,* 5 × 10⁵ cells per well) was added to each well containing 100 µL 7H9-S^{OADC} medium, serial two-fold dilutions of the peptides or controls to a final volume of 200 µL. Growth controls containing no peptide (*i.e.,* bacteria only = *B*), inhibition controls containing 50 µg/mL kanamycin and sterility controls (*i.e.,* medium only = *M*) without inoculation were also included. Plates were incubated at 37°C (32°C for *M*. *marinum*) in a humidity chamber to prevent evaporation for either 3-5 days (*M*. *smegmatis, M. abscessus*) or 10-14 days (*M. marinum, M. bovis* BCG, *M. tuberculosis* mc²6230). Then, 20 µL of a 0.025% (*w*/*v*) resazurin solution was added to each well, and the plates were incubated at 37° C for color change from blue to pink or violet and for a reading of fluorescence units (FU). Fluorescence corresponding to the resazurin reduction to its metabolite resorufin was quantified using a Tecan Spark 10M multimode microplate reader (Tecan Group Ltd, France) with excitation at 530 nm and emission at 590 nm. For fluorometric MIC determinations, a background subtraction was performed on all wells with a mean of *M* wells. Relative fluorescence units were defined as: RFU% = (test well FU/mean FU of *B* wells) × 100. MIC values were determined by fitting the RFU% sigmoidal dose-response curves in Kaleidagraph 4.2 software (Synergy Software). The lowest peptide concentrations inhibiting 90% of growth were defined as the MIC. Isoniazid (INH), amikacin (AMK), imipenem (IPM) and cefoxitin (FOX) were used as reference drugs.

The results of antimicrobial activity of Michelicin are recorded in Table IV.

**Table IV**

| | **Michelicin (MIC in µM)** | **Alvinellacin (MIC in µM)** | **Arenicin (MIC in µM)** |
|---|---|---|---|
| **Bacterial strains** | | | |
| *Salmonella enterica* (CIP 80.39) | 0.625-1.25 | 0.625-1.25 | 1.25-2.5 |
| *Escherichia coli* (ATCC 8739) | 0.625-1.25 | 1.25-2.5 | 2.5-5 |
| *E. coli* carbapenem- resistant (DSMZ 22314) | 0.625-1.25 | 0.625-1.25 | 2.5-5 |
| *E. coli* (D31 Pasteur) | 0.15-0.31 | 0.31-0.62 | 0.62-1.25 |
| *Staphylococcus aureus* (ATCC 6538P*)* | 2.5-5 | 2.5-5 | 1.25-2.5 |
| *S. aureus* (Pasteur) | 2.5-5 | 1.25-2.5 | 1.25-2.5 |
| *S. aureus* methicillin resistant USA300 (ATCC BAA-1717) | 5-10 | 2.5-5 | 5-10 |
| *Pseudomonas aeruginosa* (ATCC 9027) | 1.25-2.5 | 1.25-2.5 | 0.625-1.25 |
| *P. aeruginosa* Fluoroquinolone resistant (CIP 107398) | 2.5-5 | 1.25-2.5 | 1.25-2.5 |
| *Acinetobacter baumannii* (CIP 103572) | 1.25-2.5 | 1.25-2.5 | 1.25-2.5 |
| *A. baumannii* antibiotic resistant (CIP 110431) | 0.625-1.25 | 0.625-1.25 | 20-40 |
| *Bacillus subtilis* (ATCC 6633) (MIC Nisin < 0.09 µg/mL) | 0.625-1.25 | 0.625-1.25 | 0.625-1.25 |
| *B. subtilis* nisin resistant (DSMZ 347) MIC (Nisin 3 µg/mL) | 0.625-1.25 | 0.625-1.25 | 0.625-1.25 |
| *B. megaterium* | 0.03-0.07 | 0.15-0.31 | 0.07-0.15 |
| *Klebsiella pneumoniae carbapenem-resistant (*DSMZ 26371) | 0.625-1.25 | 1.25-2.5 | 1.25-2.5 |
| *Enterococcus faecalis* Vancomycin-resistant (DSMZ 13591) | >80 | 20-40 | 1.25-2.5 |
| *Clostridium perfringens* (ATCC 13124*)* | 1.25-2.5 | 2.5-5 | 0.625-1.25 |
| *Helicobacter pylori (ATCC 43504)* | 1.25-2.5 | 1.25-2.5 | 1.25-2.5 |
| *Mycobacterium tuberculosis* mc²6230 | 12.3-13.0 | 16.0-21.5 | 7.1-12.2 |
| *M. bovis* BCG Pasteur | 9.4-9.8 | 12.0-21.4 | 6.7-7.3 |
| *M. abscessus* R (CIP104537^{T}R*)* | 10.5-12.5 | 17.0-17.8 | 13.0-13.9 |
| *M. smegmatis* mc²155 | 0.57-2.15 | ND | 0.7-10 |
| *Vibrio alginolyticus* | 0.07-0.15 | 0.07-0.15 | 0.15-0.31 |
| *V. alginolyticus* (MPV19 IFREMER) | 0.07-0.15 | 0 .07-0.15 | 0.15-0.31 |
| *V. diabolicus (HE 800)* | 2.5-5 | 2.5-5 | 1.25-2.5 |
| *Burkholderia cepacia (DSM 7288)* | 80 | 10 | 25 |
| *Serratia marcescens* | 12.5-25 | >50 | >50 |

The results of Table IV show that Michelicin is as active as Arenicin and Alvinellacin (even more active on some strains). Further Michelicin is also active on various resistant strains.

### 3) Antimicrobial activity of variant peptides

Further, the biological activity of V1 to V10 peptides was also assessed on *Vibrio alginolyticus, Pseudomonas aeruginosa* (a human pathogenic agent), *M. smegmatis* and *M. tuberculosis,* no pathogenic and pathogenic bacteria, respectively and compared to the biological activity of Michelicin. The same experiments as described in 1) were performed. The results are shown in Table V.

**Table V**

| | *V. alginolyticus* MIC in µM | *P. aeruginosa* MIC in µM | *M. smegmatis* mc²155 MIC in µM | *M. tuberculosis mc²6230* MIC in µM |
|---|---|---|---|---|
| Michelicin | 1.25-2.5 | 2.5-5 | 0.57-2.15 | 12.3-13.0 |
| V1 | 0.125-0.312 | 2.5-5 | 1.15-4.21 | 29.8-30.2 |
| V2 | 0.312-0.625 | 2.5-5 | 0.72-2.95 | 19.7-30.0 |
| V3 | 0.625-1.25 | 2.5-5 | 0.64-4.66 | 22.9-29.6 |
| V4 | 0.312-0.625 | 2.5-5 | 1.49-4.15 | 7.5-7.9 |
| V5 | 0.625-1.25 | >20 | 3.06-8.40 | 5.3-7.9 |
| V6 | 0.125-0.312 | >5 | 1.27-2.96 | >30 |
| V7 | 0.125-0.312 | 2.5-5 | 9.22-13.1 | >30 |
| V8 | 0.125-0.312 | 1.25-2.5 | 0.72-3.92 | 7.3-11.9 |
| V9 | 0.125-0.312 | 1.25-2.5 | 1.02-3.31 | 5.1-9.1 |
| V10 | 0.125-0.312 | 1.25-2.5 | 0.21-2.82 | 12.3-13.1 |

The results of Table V show that some variants are even more efficient as antimicrobial agents than Michelicin.

### 4) Antimicrobial activity on biofilm

Biofilms formed by colonies of strains are complex systems and often resist to antimicrobial agent whereas the same strains but not forming a biofilm can be easily destroyed by the same antimicrobial agent.

### BIOFILM FORMATION ASSAYS

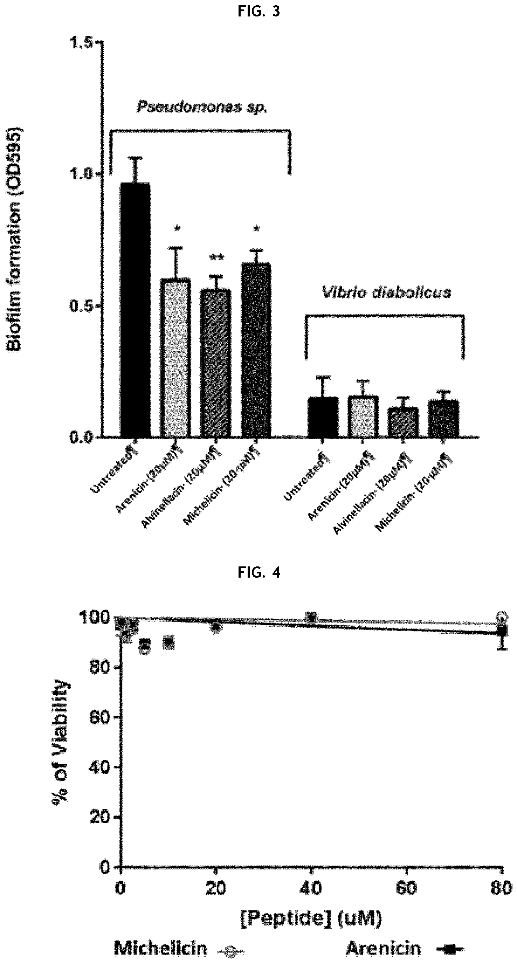

Overnight cultures of *V. diabolicus* and *Pseudomonas sp* (37° C, 200 rpm) were diluted to an OD₆₀₀ of 0.1 in MHB medium and 200 µL aliquots of the suspensions were introduced into the wells of a 96 microtiter plates (Nunc). Plates were then incubated at 37° C without shaking for 48 h. After removal of the standing culture, the wells were washed with MHB medium and Michelicin, Arenicin and Alvinellacin were added, respectively, at a final concentration of 20 µM in MHB. The treatment was performed during 2 h at 37°C. Wells were then washed three times with distilled water and the remaining attached bacteria were fixed with 99% methanol per well for 20 min at room temperature. Afterward, methanol was removed and plates were air-dried. Biofilms were stained with 300 µL of 1% crystal violet (v/v) for 20 min at room temperature, before the residual stain was removed. After washing, the crystal violet bound to the adherent cells was solubilized with 300 µL of 100% ethanol and absorbance was measured at 595 nm (TECAN, sunrise). Sterile medium served as negative control. The results are shown in Fig. 3. It is clear on the view of Fig. 3 that Michelicin has a greater antimicrobial activity on a biofilm of *Pseudomonoas sp* strains than Alvinellacin. For *Pseudomonas sp,* OD₅₉₅ is equal to 0.6 when treated with Michelicin, equal to 0.55 when treated with Arenicin and equal to 0.65 when treated with Alvinellacin. The OD₅₉₅ for untreated culture is equal to 0.95.

### 5) Effect of temperature on antimicrobial activity

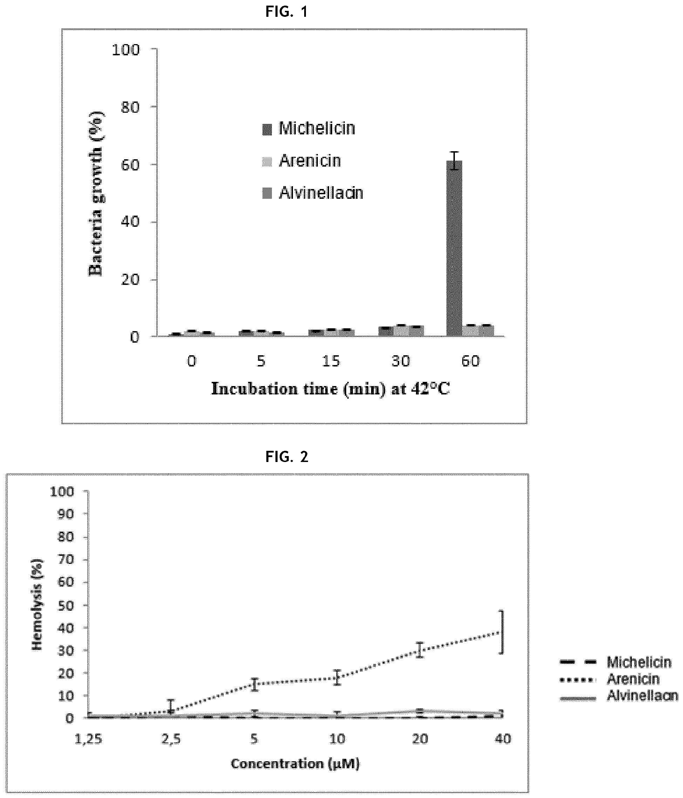

The effects of temperature on the activity of Michelicin against *Vibrio alginolyticus* were determined by inhibition zone assays. Test strain of *Vibrio alginolyticus* was grown to 0.4 OD₆₀₀ at 30°C in MHB (Mueller Hinton Broth, ROTH). A total of respectively 400 µL and 600 µL of the cell suspension were inoculated into either 50 mL of preheated MHA (at about 65°C). The medium was rapidly mixed and poured into the Petri dish (90 x 90 mm Greiner, Dominique DUTSCHER). Ten µL of AMPs at five times the MIC were filed on the Petri dish after 0, 5, 15, 30 and 60 min incubation at 90°C. After incubation at 30°C for 16-18 h, the zones of growth inhibition were measured. The results are shown in Fig. 1. Fig. 1 shows the growth of *Vibrio alginolyticus vs.* time (in min) at 90°C in presence of Michelicin, Arenicin and Alvinellacin, respectively. It appears that Michelicin is quite less thermostable than Arenicin or Alvinellacin. However, according to Fig. 1, it is obvious that Michelicin can be used as a food preservative even for pasteurized products since pasteurization is not performed for 60 minutes but rather for 5-10 min. Moreover, pasteurization is performed at a temperature from 62 to 88°C (62°C and 88°C included).

### 6) Biological activity of Michelicin with only one/no disulphide bridge

Biological (antimicrobial) activity of Michelicin (which comprises two disulfide bridges formed between four cysteines) synthetized with (A) or without (B) a stepwise formation of the desired disulfide pattern, was also determined. An analogue of Michelicin which has the same sequence as Michelicin except for the cysteine residues that are replaced by a neutral analogue of cysteine (i.e. Michelicin-OH 2343 (Abu-3-6-11-15)) was synthesized. Abu means 2-aminobutanoic group. This analogue is devoid of disulfide bridges. This analogue of Michelicin is called Michelicin no SS in Table VI. Table VI shows the MIC in µM determined with the modified Michelicin and the analogues thereof on several bacteria.

**Table VI**

| | **Folded Michelicin (MIC in µM)** | **Linear unfolded analogue of Michelicin (MIC in µM) B** | **Michelicin no SS (MIC in µM) ABU form** |
|---|---|---|---|
| **Bacterial strains** | | | |
| *Vibrio alginolyticus* | 0.625 | 0.625 | 0.625 |
| *Vibrio diabolicus* | 2.5 | 2.5 | >80 |
| *Escherichia coli* | 1.25 | 0.625 | |
| *Bacillus subtilis Nisin resistant* | 1.25 | 1.25 | 5 |
| *Pseudomonas aeruginosa Fluoroquinolone resistant* | 2.5 | 2.5 | >80 |
| *Staphylococcus aureus* | 2.5 | | 80 |
| *S. aureus Methicillin resistant (MRSA)* | 5 | 20 | >80 |
| *Mycobacterium smegmatis* | 2.15 | 1.25 | 20 |
| *Helicobacter pylori 21031* | 2 | | 40 |
| *Clostridium perfringens* | 1.25 | | >80 |
| *Salmonella enterica* | 0.625 | | 80 |
| *Acinetobacter baumannii* | 1.25 | | 2.5 |

The Michelicin (B) can be produced at a lower cost than Michelicin (A). Surprisingly, analogues of Michelicin have also antimicrobial properties.

### IV. Cytotoxicity

The effect of the peptides of the invention on haemolysis and human cells was also studied.

### 1) Haemolytic assay

The hemolytic activity of the peptides was determined using fresh human erythrocytes from healthy donors. Blood was centrifuged and the erythrocytes were washed three times with a phosphate-buffered saline (PBS: 35 mM phosphate buffer/150 mM NaCl, pH 7.4). Peptides were added to the erythrocyte suspension at a final concentration ranging from 1.2 to 40 µM in a final volume of 100 µL containing 8% human erythrocytes. Samples were incubated at 37° C for 60 min. After incubating the mixtures were centrifuged at 1,500 rpm for 10 min. The release of hemoglobin was monitored by measuring the absorbance (Abs) of the supernatant at 405 nm. Hemolytic rates of 0 and 100% were determined in PBS and 0.1% Triton X-100, respectively. The percentage of hemolysis was calculated by employing the following equation: Percentage hemolysis = [(Abs_{414 nm} in the compound solution-Abs_{414 nm} in PBS)/(Abs_{405 nm} in 0.1% Triton X-100-Abs_{405 nm} in PBS)]x100. The results are shown in Fig. 2.

As shown in Fig. 2, the effect of Michelicin on hemolysis is far less than the effect of Arenicin. It is also less than the effect of Alvinellacin. Accordingly, Michelicin can be used through intravenous injection, for example, since it has no toxic effect on blood.

### 2) Human cell culture and cytotoxicity studies

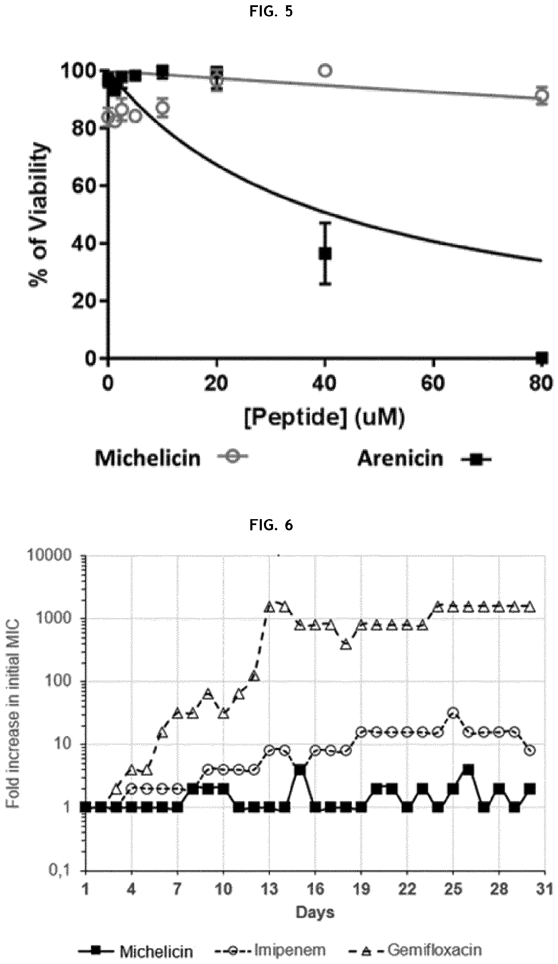

The toxicity of Michelicin on human cells was tested as previously described (Borie et al., 2017; Oyama et al., 2017). Briefly, human cells were cultured in Dulbecco's modified essential medium (DMEM) supplemented with 10% fetal calf serum (FCS), 1% L-glutamine and 1% antibiotics (all from Invitrogen). Cells were routinely grown in onto 25 cm² flasks maintained in a 5% CO₂ incubator at 37°C. For toxicity assay, cells grown on 25 cm² flasks were detached using trypsin-EDTA solution (Thermofisher) and seeded into 96-well cell culture plates (Greiner Bio-one) at approximately 10⁴ cells per well (counted using Mallasez's chamber). The cells were grown at 37°C in a 5% CO₂ incubator until they reached confluence (approximately 48-72 h of seeding). Wells were then aspirated and increasing concentrations of AMP were added to the cells and incubated for a further 48 h at 37°C in a 5% CO₂ incubator. The wells were then emptied, and cell viability was evaluated using resazurin based *in vitro* toxicity assay kit (Sigma-Aldrich) following manufacturer's instructions. Briefly, resazurin stock solution was diluted 1: 100 in sterile PBS containing calcium and magnesium (PBS++, pH 7.4) and emptied wells were filled with 100 µL of the resazurin diluted solution. After 4 h incubation at 37°C with the peptide treated cells, fluorescence intensity was measured using microplate reader (SynerMix, Bioteck) (excitation wavelength of 530 nm / emission wavelength of 590 nm). The fluorescence values were normalized by the controls and expressed as percentage of cell viability. The IC₅₀ values of tested AMP on cell viability (i.e. the concentration of peptides causing a reduction of 50% of the cell viability) were calculated using GraphPad^{®} Prism 7 software. Two cell lines were tested: Human Bronchial Epithelial Cell Line (BEAS; ATCC CRL-9609) and Human umbilical vein endothelial cell (HUVEC). Results are shown in Fig. 4 and Fig. 5.

On the view of Fig. 4, Michelicin is less cytotoxic than Arenicin, particularly at concentrations equal or higher than 40 µM. On BEAS cell lines, the % of viability is equal to 100 with 80 µM of Michelicin and equal to 95% with 80 µM of Arenicin. On HUVEC cell line, the % of viability is equal to 95% with 80 µM of Michelicin and 40% with 80 µM of Arenicin (see Fig. 5). Further, hemolytic and cytotoxic activities towards human endothelial cells (HUVEC cells), human airway epithelial cells (BEAS cells) or human lung fibroblasts (IMR90 cells (ATCC CCL-186)) were not observed even at doses >80 µM. This lack of toxicity gives a very broad therapeutic window.

### V. Serial passage/resistance assay- resistance test

### 1) Resistance tests on Pseudomonas aeruginosa

*In vitro* evaluation of the potential for bacteria (*P. aeruginosa* in the present case) to develop resistance to Michelicin or comparator antibiotics (Imipenem and Gemifloxacin) was performed as previously described (Oyama et al., 2017). Briefly, on Day 1, bacteria were subjected to microbroth dilution susceptibility testing performed using a standard doubling-dilution series of AMP concentrations as described for MIC determination. Cultures from the highest concentration that supported growth were diluted 1: 1000 in MHB and used to provide the inoculum for the next passage day. This process was continued for 25-30 days. The last growing wells (corresponding to ½ of the MIC value) were used to prepare next day experiments allowing to test the chronic exposure to sub-lethal doses of antibiotics during 30 consecutive days. The results are shown in Fig. 6. The fold increase in initial MIC is not relevant for all the AMP whereas it is relevant for Imipenem and Gemifloxacin. For both antibiotics, the fold increase is continuously increasing *vs* time. Resistance induction is evident only for reference antibiotics with an important increase in their MIC values (up to 20 to 1000-fold). Michelicin does not induce any resistance in *P. aeruginosa* within a treatment of 30 days.

### 2) Membrane permeabilization assay

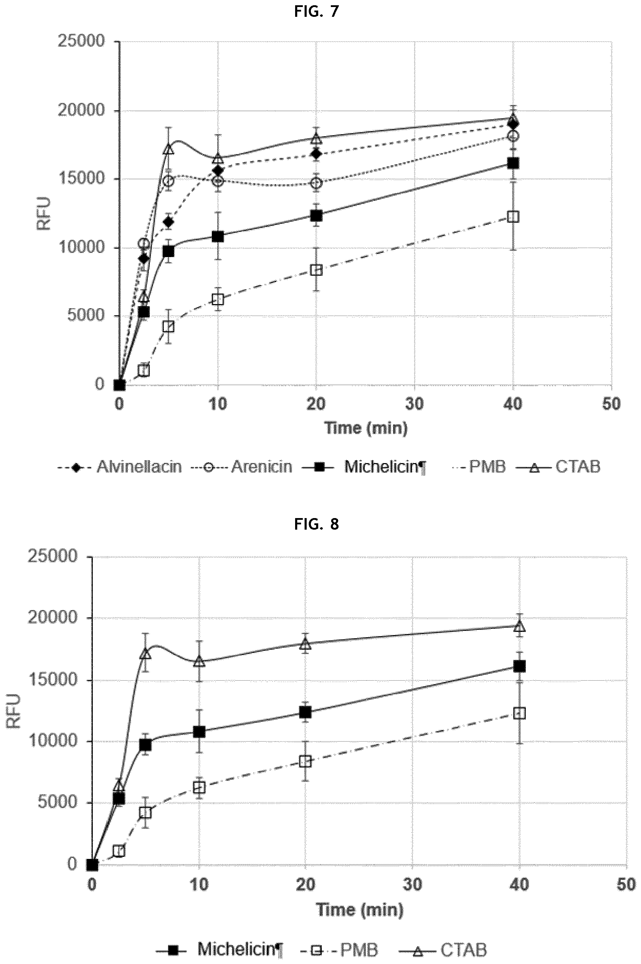

Several tests were performed to determine the mechanism of the antimicrobial activity of the peptides of the invention. Most of the cationic AMP can increase the permeability of the bacterial membrane through pore-forming activity. We evaluated the ability of Michelicin to cause pore-formation using *P. aeruginosa* as model bacteria. Permeabilization of the bacterial membrane by Michelicin was evaluated using the cell-impermeable DNA probe propidium iodide (PI) (Di Pasquale et al., 2010; Oyama et al., 2017). PI can interact with DNA/RNA, leading to increase in fluorescence of the molecule. The bacterial membrane is impermeable to PI preventing its interaction with intracellular DNA/RNA. Thus, fluorescence of PI (expressed as RFU: relative fluorescence unit) only increases if membrane integrity is compromised through pore-formation. We measured the time-dependent effect of our peptide and other molecules on PI fluorescence at 37°C, all molecules being tested at 3 times their MIC. Briefly, logarithmic phase bacterial suspensions were prepared by diluting over-night cultures in fresh Mueller-Hinton (MH) broth (1 in 10 dilution), incubated 3 h at 37°C, 200 rpm, and pelleted by centrifugation for 5 min at 3000 *g*. Bacterial cell pellet was then resuspended in sterile phosphate buffer saline (PBS) at 10⁹ bacteria/mL. Propidium iodide was then added to the bacterial suspension at a final concentration of 60 µM. One hundred µL of the suspension were transferred into black 96-well plates already containing 100 µL of serially diluted AMP in PBS. Kinetics of fluorescence variations (excitation at 530 nm / emission at 590 nm) were recorded using a microplate reader (SynergyMx, Bioteck) over 80 min period at 37°C. Cetyl trimethylammonium bromide (CTAB) (at 300 µM) served as positive control giving 100% permeabilization. The permeabilization effect of the tested compound (*i.e.,* Michelicin, Alvinellacin, Arenicin, and control, PMB or CTAB) was expressed as percentage of total permeabilization caused by CTAB. Results are shown in Fig. 7 and Fig. 8.

Data show that Michelicin can increase bacterial membrane permeability. Its action is similar to the one of CTAB (Cetrimonium bromide), a cationic detergent and higher to polymyxin B (PMB) another cationic AMP. Indeed, Michelicin and CTAB cause pore-formation very fast with 25-30% permeabilization at 2.5 min of incubation, whereas PMB acts slower with only 2-5% permeabilization at that time of incubation, showing that Michelicin is more active than PMB in term of pore-formation.

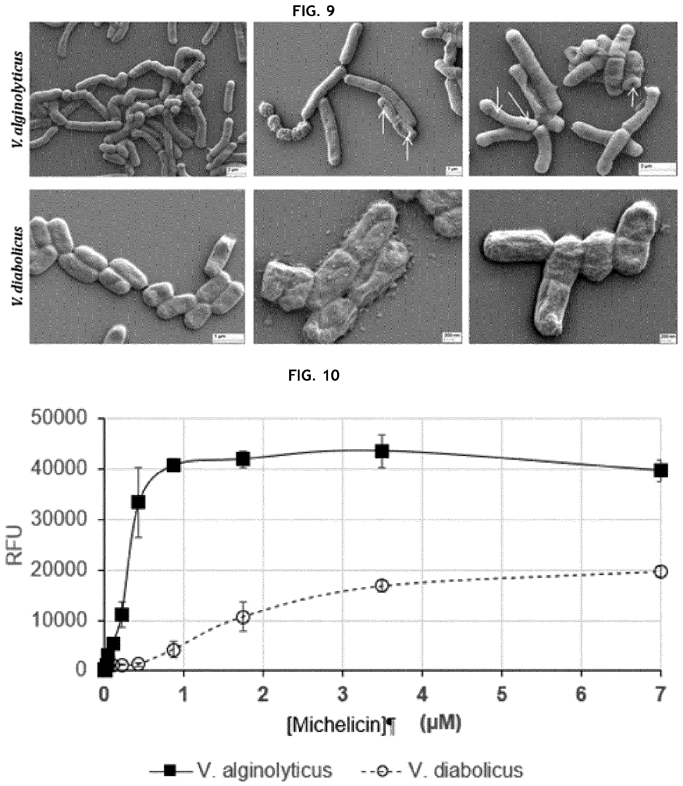

These results are confirmed by Fig. 9. In Fig. 9, it is clear that Michelicin and Alvinellacin are able to damage *V. alginolyticus* and *V. diabolicus* membrane. The arrows show pore formed through the membrane. For *V. diabolicus,* despite the results of Fig. 3, is seems that Michelicin has a pore-forming activity on these bacteria. Fig. 9 shows that bacteria collapse, their membrane becoming blurred due to pore-forming degradation.

Evaluation of the permeabilization of the bacterial membrane of *V*. *alginolyticus* and *V. diabolicus* by the propidium iodide assay (Fig. 10) confirmed pore-forming activity of Michelicin on these bacteria with a preferential/higher effect of Michelicin on *V. alginolyticus* compared to *V. diabolicus,* in accordance with its MIC values on the two strains (0.07-0.15 versus 2.5-5 µM, respectively).

### BIBLIOGRAPHIC REFERENCES

Knight, S. D., Presto, J., Linse, S., & Johansson, J. (2013). The BRICHOS domain, amyloid fibril formation, and their relationship. Biochemistry, 52(43), 7523-7531. Boidin-Wichlacz, C., Jollivet, D., Papot, C., Roisin, L., Massol, F., & Tasiemski, A. (2021). Genetic diversification and life-cycle of the polychaete Capitella spp. from the English Channel: evidence for sympatric cryptic species and alternative reproductive strategies. Marine Biology, 168(12), 1-20.
Rowe et al, eds Handbook of Pharmaceutical Excipients, 7th edn, London, pharmaceutical press 2012

## Claims

1. An isolated peptide, a variant or a chemical analogue thereof, wherein the isolated peptide and the variant thereof are selected from:
a) peptide consisting in any one of sequences selected from SEQ ID NO: 2 to SEQ ID NO: 12;
b) peptide comprising a sequence selected from any one of SEQ ID NO: 2 to SEQ ID NO: 12;
c) peptide as defined in a) or b) and comprising a modifying group of its C-terminal carboxyl group and/or of its N-terminal amine group;
d) peptide having at least 80%, particularly at least 90% and more particularly at least 95% identity with peptide as defined in a), b) or c) and
e) retro and retro-inverso peptide of peptides as defined in a) to d),
wherein the chemical analogues are selected from:
f) a chemical analogue consisting in any one of sequences selected from SEQ ID NO: 13 to SEQ ID NO: 19;
g) a chemical analogue comprising a sequence selected from any one of SEQ ID NO: 13 to SEQ ID NO: 19;
h) a chemical analogue as defined in f) or g) and comprising a modifying group of its C-terminal carboxyl group and/or of its N-terminal amine group,
i) a chemical analogue having at least 80%, particularly at least 90% and more particularly at least 95% identity with analogues as defined in f), g) or h);
j) retro and retro-inverso peptide of peptides as defined in f) to i),
said peptide and variants as defined in a) to e) and said chemical analogues as defined in f) to j) having an antimicrobial activity.

2. An isolated peptide, variants or chemical analogues thereof according to claim 1, **characterized in that** it comprises at least two cysteines and particularly four cysteines.

3. An isolated peptide, variants or chemical analogues thereof according to any one of claims 1 or 2, wherein said peptide, variants or chemical analogues thereof is a L-peptide.

4. An isolated peptide, variants or chemical analogues thereof according to any one of claims 1 to 3, wherein said peptide, variants or chemical analogues thereof is a non-folded peptide.

5. An isolated peptide, variants or chemical analogues thereof according to any one of the claims 1 to 4, wherein said N-terminal modifying group and/or said C-terminal modifying group are selected independently from each other from acetyl group; formyl group; palmitoyl group; fatty acids groups, particularly myristoyl group, pyroglutamyl group; urea; 7-amino-4-methylcoumarinyl; carbamate; sulphonamide; alkylamine; benzoyl; benzyloxycarbonyl; dye; labels, particularly biotin or fluorescent molecules; NH₂; -O-alkyl,particularly *O*-methyl, *O*-hexyl, *O*-decyl, *O*-tetradecanyl, *O*-methyl and *O*-ethyl;H; NHC₆H₆NO₂(*para*); 7-amino-4-methylcoumarinyl; NHNH₂; NHOH; CH₂Cl; (CH₂)₂NH₂; NHCH₃; NHCH₂CH₃; alkyl and particularly methyl.

6. An isolated peptide, variants or chemical analogues thereof according to any one of claims 1 to 5, wherein at least one of cysteines and preferably all cysteines are replaced by an analogue of cysteine and particularly by 2-aminobutanoic group and particularly, said isolated peptide is of sequence SEQ ID NO 2, wherein at least one of cysteines and preferably all cysteines are replaced by an analogue of cysteine and particularly by 2-aminobutanoic group.

7. An isolated nucleic acid molecule encoding a peptide, variants or chemical analogues thereof of any one of claims 1 to 6, in particular a nucleic acid molecule encoding a peptide, variants or chemical analogues thereof of SEQ ID NO: 2 to SEQ ID NO: 19 and particularly, the nucleic acid molecule is of sequence SEQ ID NO: 20.

8. A recombinant nucleic acid construct comprising the nucleic acid molecule of claim 7 operably linked to an expression vector.

9. A host cell comprising the recombinant nucleic acid construct of claim 8, said host cell being particularly from *E. coli* or *P. pastoris.*

10. A pharmaceutical composition comprising:
- at least one selected from: the isolated peptide, variants or chemical analogues thereof according to any one of claims 1 to 6, the isolated nucleic acid molecule according to claim 7, the recombinant nucleic acid construct according to claim 8 and the host cell according to claim 10, and
- a pharmaceutically acceptable excipient.

11. The isolated peptide, variants or chemical analogues thereof according to any one of claims 1 to 6, the isolated nucleic acid molecule according to claim 7, the recombinant nucleic acid construct according to claim 8, the host cell according to claim 9 or the pharmaceutical composition according to claim 10 for use as drug.

12. The isolated peptide, variants or chemical analogues thereof according to any one of claims 1 to 6, the isolated nucleic acid molecule according to claim 7, the recombinant nucleic acid construct according to claim 8, the host cell according to claim 9 or the pharmaceutical composition according to claim 10, for use as an antimicrobial agent, particularly an antibiotic for treating a subject in need thereof and more particularly as an antimicrobial agent for treating an infection and/or disease caused by at least one bacterium selected from:
i) Gram negative bacteria, in particular selected from the group of genius consisting of:
a) *Acinetobacter, particularly Acinetobacter baumannii (*CIP103572) and more particularly *A. baumannii resistant strain (CIP 110431);*
b) *Salmonella, particularly Salmonella enterica (CIP 80.39);*
c) *Escherichia, particularly Escherichia coli* and more particularly *E. coli (ATCC 8739), E. coli carbapenem resistant strain (DSMZ 22314) and E. coli (D31);*
*d) Pseudomonas, particularly Pseudomonas aeruginosa (ATCCC CRM-9027) and more particularly P. aeruginosa fluoroquinolone resistant strain (CIP 107398);*
*e) Klebsiella, particularly Klebsiella pneumoniae* and more particularly *K. pneumoniae carbapenem resistant strain (DSMZ 26371);*
*f) Helicobacter, particularly Helicobacter pylori (ATCC 43504);*
*g) Vibrio, particularly Vibrio alginolyticus and Vibrio diabolicus (HE 800);*
*h) Burkholderia, particularly Burkholderia cepacia (DSM7288), and*
*i) Serratia, particularly Serratia marcescens;*
ii) *Gram positive bacteria, in particular* selected from the group of genius consisting of:
a) *Staphylococcus, particularly Staphylococcus aureus (ATCC 6538P), S. aureus (Pasteur) and more particularly S. aureus (MR, Methicilin Resistant) USA300 (ATCC BAA-1717);*
*b) Bacillus, particularly Bacillus subtilis (ATCC 6633) and more particularly B. subtilis Nisin resistant (DSM 347) and B. megaterium;*
*c) Enterococcus, particularly Enterococcus faecalis VRE (Vancomycin Resistant) (DSM 13591);*
*d) Clostridium, particularly Clostridium perfringens (ATCC 13124), and*
*iii) Mycobacterium species, in particular: M. tuberculosis and more particularly, M. tuberculosis* mc²6230, *M. bovis* BCG *Pasteur, M. abscessus, M. smegmatis, M. ulcerans and M. marinum.*

13. The isolated peptide, variants or chemical analogues thereof according to any one of claims 1 to 6, the isolated nucleic acid molecule according to claim 7, the recombinant nucleic acid construct according to claim 8, the host cell according to claim 9 or the pharmaceutical composition according to claim 10, for use according to claim 11 or claim 12, wherein said subject is a mammal, preferably a human being, more preferably a human being suffering from a disease, preferably pulmonary or skin disease.

14. The isolated peptide, variants or chemical analogues thereof according to any one of claims 1 to 6, the isolated nucleic acid molecule according to claim 7, the recombinant nucleic acid construct according to claim 8, the host cell according to claim 9 or the pharmaceutical composition according to claim 10, for use according to claim 11 or claim 12, wherein said subject is a fresh or marine animal, preferably selected from the group consisting of:
(i) fishes, preferably carps, cyprinids, and salmonids;
(ii) shellfishes, preferably oysters, clams, cockles, ark shells, scallops and mussels, and
(iii) crustaceans, preferably crabs, lobsters, shrimps and prawns.

15. Use of the isolated peptide, variants or chemical analogues thereof according to any one of claims 1 to 6, the isolated nucleic acid molecule according to claim 7, the recombinant nucleic acid construct according to claim 8 or the host cell according to claim 9 or the pharmaceutical composition according to claim 10 as preservative for:
(i) *in vitro* treating of a mammal semen, and
(ii) treating plants, particularly aquatic plants, preferably algae or phytoplankton.
